# EUROPÄISCHE PATENTANMELDUNG

(11) **EP 4 684 892 A1**
(43) Veröffentlichungstag der Anmeldung: **28.01.2026**
(21) Anmeldenummer: 25179847.6
(22) Anmeldetag: 30.05.2025
(51) Int. Cl.: B09B 1/00, C08J 11/00, B29B 17/00, C07C 67/333, C07C 69/54, C07C 67/475, B01J 8/18, F23C 10/00, B29B 17/02, C10B 49/22, C10B 53/07, C07C 4/22, C08J 11/12

(54) **VERFAHREN UND VORRICHTUNG ZUM WIEDERAUFBEREITEN VON VERBUNDKUNSTSTOFFTEILEN**

(30) Priorität: 18.06.2024 DE 102024205621
(71) Anmelder: BLANCO GmbH + Co KG, 75038 Oberderdingen (DE)
(72) Erfinder: Hajek, Andreas, 76356 Weingarten (DE)
(74) Vertreter: Ullrich & Naumann PartG mbB

(57) **Zusammenfassung**

Die Erfindung betrifft ein Verfahren zum Wiederaufbereiten von Verbundkunststoffteilen, beispielsweise aus aushärtbaren Gießmassen hergestellte Formteile, wie Sanitärbecken, umfassend zumindest einen Füllstoff und zumindest ein Polymer, insbesondere Polymethylmethacrylat, mit den Schritten:
- Zerkleinern zumindest eines Verbundkunststoffteils zu einem Aufgabegut,
- Zuführen des Aufgabeguts in einen Wirbelschichtreaktor mit zumindest einem Wirbelgut,
- Depolymerisieren des zumindest einen Polymers des Aufgabeguts mittels Pyrolyse zu einem Pyrolyseprodukt, insbesondere umfassend Methylmethacrylat,
- Trennen des Füllstoffs von dem Pyrolyseprodukt, wobei der abgetrennte Füllstoff und das Wirbelgut in Material und/oder Größenverteilung im Wesentlichen ähnlich, insbesondere gleich, ausgebildet sind,
- Abführen eines Fluidstroms umfassend das Pyrolyseprodukt aus dem Wirbelschichtreaktor, und
- Abführen zumindest eines Teils des abgetrennten Füllstoffs gemeinsam mit zumindest einem Teil des Wirbelguts aus dem Wirbelschichtreaktor.

## Beschreibung

Die Erfindung betrifft ein Verfahren zum Wiederaufbereiten von Verbundkunststoffteilen, beispielsweise aus aushärtbaren Gießmassen hergestellte Formteile, wie Sanitärbecken, umfassend zumindest einen Füllstoff und zumindest ein Polymer, insbesondere Polymethylmethacrylat.

Die Erfindung betrifft weiter eine Vorrichtung zum Wiederaufbereiten von Verbundkunststoffteilen.

Die Bedeutung des Recyclings von Verbundkunststoffen wächst aufgrund der steigenden Menge an Kunststoffabfällen und der zunehmenden Bestrebungen, Ressourcen zu schonen und Umweltbelastungen zu reduzieren. Durch Recycling sollen Materialien wiederverwertet werden können und der Bedarf an neuen Rohstoffen verringert werden, was sowohl in ökologischer als auch ökonomischer Hinsicht Vorteile mit sich bringen soll.

Aus der WO 00/17149 A1 ist ein Verfahren zum Wiederaufbereiten von Polymethylmethacrylat bekannt geworden, wobei das Polymermaterial in einem Reaktor mit heißem mechanisch verwirbeltem Feststoff als Wirbelgut in Kontakt gebracht und dabei depolymerisiert wird. Dabei entstehende Dämpfe werden abgeleitet und kondensiert. Das dadurch erhaltene Pyrolyseöl enthält Methylmethacrylat, was zur erneuten Herstellung von Polymeren verwendet werden kann. Im Rahmen des Verfahrens wird das heiße Wirbelgut kontinuierlich an einem Ende des Reaktors zugeführt und am anderen Ende wieder ausgetragen.

Bei Anwendung dieses Verfahrens zum Wiederaufbereiten von Verbundkunststoffen, die neben Polymeren meist einen Füllstoff, beispielsweise Kohlenstofffasern oder anorganisches Material, enthalten, ist jedoch nachteilig, dass der abgetrennte Füllstoff gemeinsam mit dem Wirbelgut aus dem Reaktor ausgetragen wird und daher aufwendig in einem zusätzlichen Prozessschritt von dem Wirbelgut abgetrennt werden muss.

Eine Aufgabe der vorliegenden Erfindung ist es daher ein Verfahren und eine Vorrichtung zum Wiederaufbereiten von Verbundkunststoffteilen anzugeben, welche eine einfache und effiziente Wiederaufbereitung von Verbundkunststoffteilen ermöglichen.

Eine weitere Aufgabe der vorliegenden Erfindung ist es, ein alternatives Verfahren und eine alternative Vorrichtung zum Wiederaufbereiten von Verbundkunststoffteilen anzugeben.

In einer Ausführungsform löst die vorliegende Erfindung die vorstehend genannten Aufgaben mit einem Verfahren zum Wiederaufbereiten von Verbundkunststoffteilen, beispielsweise aus aushärtbaren Gießmassen hergestellte Formteile, wie Sanitärbecken, umfassend zumindest einen Füllstoff und zumindest ein Polymer, insbesondere Polymethylmethacrylat, mit den Schritten:
- Zerkleinern zumindest eines Verbundkunststoffteils zu einem Aufgabegut,
- Zuführen des Aufgabeguts in einen Wirbelschichtreaktor mit zumindest einem Wirbelgut,
- Depolymerisieren des zumindest einen Polymers des Aufgabeguts mittels Pyrolyse zu einem Pyrolyseprodukt, insbesondere umfassend Methylmethacrylat,
- Trennen des Füllstoffs von dem Pyrolyseprodukt, wobei der abgetrennte Füllstoff und das Wirbelgut in Material und/oder Größenverteilung im Wesentlichen ähnlich, insbesondere gleich, ausgebildet sind,
- Abführen eines Fluidstroms umfassend das Pyrolyseprodukt aus dem Wirbelschichtreaktor, und
- Abführen zumindest eines Teils des abgetrennten Füllstoffs gemeinsam mit zumindest einem Teil des Wirbelguts aus dem Wirbelschichtreaktor.

In einer Ausführungsform löst die vorliegende Erfindung die vorstehend genannten Aufgaben mit einer Vorrichtung zum Wiederaufbereiten von Verbundkunststoffteilen, beispielsweise aus aushärtbaren Gießmassen hergestellte Formteile, wie Sanitärbecken, umfassend zumindest einen Füllstoff und zumindest ein Polymer, insbesondere Polymethylmethacrylat, insbesondere mit einem Verfahren gemäß einem der Ansprüche 1 bis 14, umfassend eine Zerkleinerungseinrichtung zum Zerkleinern der Verbundkunststoffteile zu einem Aufgabegut, eine Zuführeinrichtung zum Zuführen des Aufgabeguts in einen Wirbelschichtreaktor mit zumindest einem Wirbelgut, den Wirbelschichtreaktor zum Depolymerisieren des zumindest einen Polymers des Aufgabeguts mittels Pyrolyse zu einem Pyrolyseprodukt, insbesondere umfassend Methylmethacrylat, und Trennen des Füllstoffs von dem Pyrolyseprodukt, wobei der abgetrennte Füllstoff und das Wirbelgut in Material und/oder Größenverteilung im Wesentlichen ähnlich, insbesondere gleich, ausgebildet sind, und eine Abführeinrichtung zum Abführen eines Fluidstroms umfassend das Pyrolyseprodukt aus dem Wirbelschichtreaktor und zum Abführen zumindest eines Teils des abgetrennten Füllstoffs gemeinsam mit zumindest einem Teil des Wirbelguts aus dem Wirbelschichtreaktor.

Einer der dadurch erzielten Vorteile ist, dass auf eine aufwendige Trennung der abgeführten Feststoffe in Füllstoff und Wirbelgut verzichtet werden kann. Die abgeführten Feststoffe können dem Wirbelschichtreaktor erneut zugeführt werden und/oder gesammelt werden, um für die Herstellung neuer Verbundkunststoffteile verwendet zu werden. Somit kann ein zeit- und kosteneffizienteres Verfahren bereitgestellt werden. Des Weiteren kann auf eine Trenneinrichtung als Teil der Vorrichtung verzichtet werden, wodurch ein vereinfachter Aufbau der Vorrichtung bereitgestellt werden kann.

Das Wirbelgut kann dabei von unten durch eine poröse Platte im Wirbelschichtreaktor zu einem Wirbelstrom aufgewirbelt werden, wodurch ein Wirbelbett entsteht. Dabei kann dem Wirbelschichtreaktor ein Wirbelgas zugeführt werden. Das Aufgabegut kann direkt in das heiße Wirbelbett gefördert werden, woraufhin die Depolymerisierung des zumindest einen Polymers zu dem Pyrolyseprodukt stattfinden kann. Die Reaktionszeit kann dabei im Bereich weniger Sekunden liegen. Infolge der Zersetzung des zumindest einen Polymers und der Verwirbelung wird der Füllstoff von dem Pyrolyseprodukt getrennt.

Der Begriff "in Material im Wesentlichen ähnlich" ist im weitesten Sinne zu verstehen und bezieht sich, insbesondere in den Ansprüchen, vorzugsweise in der Beschreibung auf eine prozentuale Übereinstimmung der Materialzusammensetzungen, insbesondere definiert durch die chemische Zusammensetzung und/oder die mikrostrukturellen Eigenschaften, bezogen auf die Volumen- und/oder Massenanteile von mindestens 80 %, vorzugsweise mindestens 90 %, insbesondere mindestens 95 %. In diesem Zusammenhang ist unter dem Begriff "in Material gleich" eine prozentuale Übereinstimmung von mindestens 99 % zu verstehen.

Der Begriff "in Größenverteilung im Wesentlichen ähnlich" ist im weitesten Sinne zu verstehen und bezieht sich, insbesondere in den Ansprüchen, vorzugsweise in der Beschreibung auf eine prozentuale Übereinstimmung von Partikelgrößen und/oder Partikelgrößenfraktionen bezogen auf die Volumen- und/oder Massenanteile von mindestens 80 %, vorzugsweise mindestens 90 %, insbesondere mindestens 95 %. In diesem Zusammenhang ist unter dem Begriff "in Größenverteilung gleich" eine prozentuale Übereinstimmung von mindestens 99 % zu verstehen.

Weitere Merkmale, Vorteile und weitere Ausführungsformen der Erfindung sind im Folgenden beschrieben oder werden dadurch offenbar.

Gemäß einer vorteilhaften Weiterbildung der Erfindung weist zumindest ein Teil des Aufgabeguts eine Partikelgröße von zumindest 1 µm, vorzugsweise zumindest 0,1 mm, insbesondere zumindest 1 mm, und maximal 10 mm, vorzugsweise maximal 7 mm, insbesondere maximal 5 mm, auf, insbesondere wobei das Aufgabegut dem Wirbelschichtreaktor mittels zumindest eines Schneckenförderers zugeführt wird. Dadurch ist eine besonders schnelle Depolymerisierung des Aufgabeguts möglich, wodurch die Effizienz des Verfahrens gesteigert werden kann. Mithilfe eines Schneckenförderers kann ein Verfahren mit einer kontinuierlichen Zuführung von Aufgabegut bei einer hohen Genauigkeit der Dosierung bereitgestellt werden.

Gemäß einer weiteren vorteilhaften Weiterbildung der Erfindung weist zumindest ein Teil des Aufgabeguts eine Partikelgröße zwischen 50 mm und 100 mm auf, insbesondere wobei das Aufgabegut dem Wirbelschichtreaktor über zumindest eine Doppelklappenschleuse zugeführt wird. Einer der dadurch erzielten Vorteile ist, dass der Aufwand zum Zerkleinern der Verbundkunststoffteile reduziert wird. Eine Doppelklappenschleuse ermöglicht eine einfache und schnelle Zuführung großer Mengen von Aufgabegut.

Gemäß einer weiteren vorteilhaften Weiterbildung der Erfindung beträgt eine Betriebstemperatur des Wirbelschichtreaktors zwischen 400 °C und 650 °C, vorzugsweise zwischen 425 °C und 500 °C, insbesondere 450 °C. Ein Vorteil hiervon ist, dass die meisten Polymere in diesen Temperaturbereichen depolymerisierbar sind. Eine Betriebstemperatur von 450 °C eignet sich besonders gut zum Depolymerisieren von Polymethylmethacrylat mittels Pyrolyse.

Gemäß einer weiteren vorteilhaften Weiterbildung der Erfindung wird der Wirbelschichtreaktor mithilfe zumindest eines Strahlheizrohres beheizt. Strahlheizrohre ermöglichen die Bereitstellung einer homogenen Temperaturverteilung innerhalb des Wirbelschichtreaktors, was die Reaktionsbedingungen und die Effizienz des Pyrolyseprozesses verbessert.

Gemäß einer weiteren vorteilhaften Weiterbildung der Erfindung wird das zumindest eine Strahlheizrohr zumindest teilweise mit zumindest einem Überschussgas aus der Pyrolyse betrieben, insbesondere wobei zumindest eines des zumindest einen Überschussgases von dem Fluidstrom abgeschieden wird. Dadurch wird ein besonders ressourcenschonendes Verfahren bereitgestellt.

Gemäß einer weiteren vorteilhaften Weiterbildung der Erfindung wird dem Wirbelschichtreaktor zur Erzeugung eines Wirbelstroms ein Wirbelgas, insbesondere ein Inertgas, wie beispielsweise Stickstoff, mit einem Druck zwischen 140 mbar und 180 mbar, vorzugsweise von 160 mbar, zugeführt. Somit kann ein Wirbelstrom zur homogenen Durchmischung und gleichmäßigen Temperaturverteilung im Wirbelschichtreaktor erzeugt werden. Die gleichmäßige Temperaturverteilung verhindert die Bildung von Temperaturkonzentrationen, die zu einer Zersetzung des Reaktionsmaterials führen können. Der Einsatz von Inertgasen, wie Stickstoff, verringert dabei die Wahrscheinlichkeit unerwünschter chemischer Reaktionen.

Gemäß einer weiteren vorteilhaften Weiterbildung der Erfindung weisen der Füllstoff und das Wirbelgut zumindest teilweise, insbesondere vollständig, anorganisches Material, beispielsweise Quarzsand, auf. Anorganische Materialien wie Quarzsand haben eine hohe Schmelztemperatur und weisen somit eine hohe thermische Stabilität auf. Dies ermöglicht einen Betrieb des Wirbelschichtreaktors bei hohen Temperaturen, ohne dass der Füllstoff schmilzt oder sich zersetzt. Dies würde zu Verunreinigungen des Pyrolyseprodukts führen, die aufwendig abgeschieden werden müssten.

Gemäß einer weiteren vorteilhaften Weiterbildung der Erfindung wird der Fluidstrom mit einem Druck zwischen 40 mbar und 60 mbar, vorzugsweise von 50 mbar, aus dem Wirbelschichtreaktor abgeführt. Dadurch können für den Pyrolyseprozess günstige Reaktionsbedingungen mit einem geeigneten Druck im Wirbelschichtreaktor bereitgestellt werden.

Gemäß einer weiteren vorteilhaften Weiterbildung der Erfindung wird der Fluidstrom zumindest einem Fliehkraftabscheider, insbesondere Aerozyklon, zum Abtrennen von Feststoffpartikeln zugeführt. Somit wird der durch Feststoffpartikel hervorgerufene Verschleiß der Einrichtung verringert. Ein Aerozyklon ist besonders gut geeignet zum kontinuierlichen Abtrennen von Feststoffpartikeln, was die Reinheit des abgeführten Fluidstroms erhöht.

Gemäß einer weiteren vorteilhaften Weiterbildung der Erfindung wird der Fluidstrom, insbesondere nach dem Zuführen zu dem zumindest einen Fliehkraftabscheider, zumindest einem, vorzugsweise drei, Gaswäschern zugeführt. Vorteil hiervon ist, dass gasförmige Verunreinigungen und Partikel aus dem Fluidstrom entfernt werden können. Verschmutzungen der Einrichtung, beispielsweise durch Ablagerungen, können somit vermieden werden.

Gemäß einer weiteren vorteilhaften Weiterbildung der Erfindung wird der Fluidstrom, insbesondere nach dem Zuführen zu dem zumindest einen Gaswäscher, zumindest einem Elektroabscheider zugeführt. Einer der dadurch erzielten Vorteile ist, dass besonders feine Partikel und Aerosole, die insbesondere durch den zumindest einen Gaswäscher nicht vollständig entfernt werden können, abgetrennt werden können. Verschmutzungen der Einrichtung, beispielsweise durch Ablagerungen, können somit vermieden werden.

Gemäß einer weiteren vorteilhaften Weiterbildung der Erfindung wird das Pyrolyseprodukt durch Kondensation als Pyrolyseöl von dem Fluidstrom abgeschieden. Das abgeschiedene Pyrolyseprodukt, welches im Wesentlichen monomere Reaktionsprodukte enthält, kann somit einer erneuten Verwertung zugeführt werden. Beispielsweise können neue Formteile, wie Sanitärbecken, aus aushärtbaren Gießmassen hergestellt werden. Es ist denkbar, dass das Pyrolyseöl aufgereinigt wird, um die Reinheit der aus der Depolymerisierung des zumindest einen Polymers erhaltenen, im Wesentlichen monomeren Reaktionsprodukte zu erhöhen.

Gemäß einer weiteren vorteilhaften Weiterbildung der Erfindung wird der Fluidstrom in einem Kreislauf geführt, wobei der Fluidstrom nach dem Abscheiden des Pyrolyseprodukts wieder dem Wirbelschichtreaktor zugeführt wird. Einer der dadurch erzielten Vorteile ist, dass die Material- und Energieeffizienz des Verfahrens verbessert werden. Zudem trägt die Rückführung des Fluidstroms zur Verringerung von Abfallprodukten bei, die aufwendig entsorgt werden müssen.

Weitere wichtige Merkmale und Vorteile der Erfindung ergeben sich aus den Unteransprüchen, aus den Zeichnungen und aus der dazugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungen und Ausführungsformen der vorliegenden Erfindung sind in den Zeichnungen dargestellt und werden in der nachfolgenden Beschreibung näher erläutert, wobei sich gleiche Bezugszeichen auf gleiche oder ähnliche oder funktional gleiche Bauteile oder Elemente beziehen.

Dabei zeigen
- Fig. 1: Schritte eines Verfahrens gemäß einer Ausführungsform der vorliegenden Erfindung, und
- Fig. 2: in schematischer Darstellung eine Vorrichtung gemäß einer Ausführungsform der vorliegenden Erfindung.

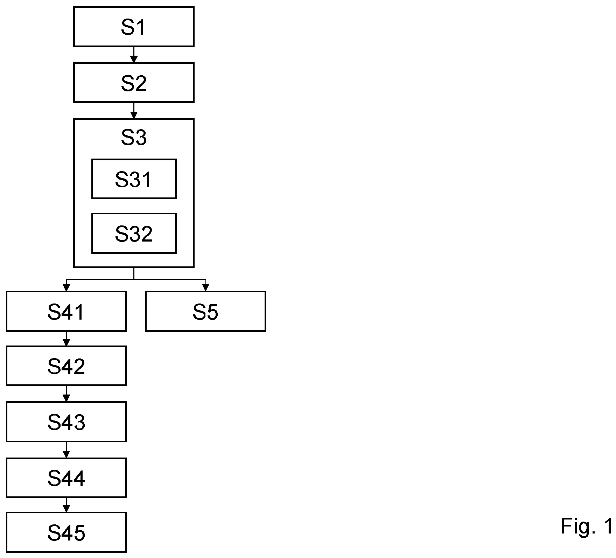

Die Figur 1 zeigt Schritte eines Verfahrens gemäß einer Ausführungsform der vorliegenden Erfindung.

Das in Figur 1 dargestellte Verfahren zum Wiederaufbereiten von Verbundkunststoffteilen wird nachfolgend in Bezug auf aus aushärtbaren Gießmassen hergestellte Sanitärbecken, umfassend einen Füllstoff und Polymere, beschrieben. Der Füllstoff umfasst dabei im Wesentlichen Quarzsand und die Polymere umfassen dabei im Wesentlichen Polymethylmethacrylat. Es wird darauf hingewiesen, dass das nachfolgend beschriebene Verfahren auch auf andere Verbundkunststoffteile, umfassend zumindest einen Füllstoff und zumindest ein Polymer, anwendbar ist.

Die Verbundkunststoffteile werden in einem ersten Schritt S1 zu einem Aufgabegut zerkleinert, welches anschließend in Schritt S2 einem Wirbelschichtreaktor mit einem Wirbelgut zugeführt wird. Das Aufgabegut wird dabei mittels zweier Schneckenförderer und/oder über eine Doppelklappenschleuse zugeführt. Zum Zuführen mittels der Schneckenförderer werden die Verbundkunststoffteile auf eine Partikelgröße von zumindest 1 µm und maximal 10 mm zerkleinert. Zum Zuführen mittels der Doppelklappenschleuse werden die Verbundkunststoffteile auf eine Partikelgröße zwischen 50 mm und 100 mm zerkleinert.

Der Schritt S3 umfasst das Depolymerisieren - Schritt S31 - der Polymere mittels Pyrolyse zu einem Pyrolyseprodukt, im Wesentlichen umfassend Methylmethacrylat, und Trennen - Schritt S32 - des Füllstoffs von dem Pyrolyseprodukt. Das Wirbelgut und der abgetrennte Füllstoff sind dabei in Material und Größenverteilung im Wesentlichen ähnlich ausgebildet. Im Rahmen dieses Prozesses kann das Wirbelgut von unten durch eine poröse Platte im Wirbelschichtreaktor zu einem Wirbelstrom aufgewirbelt werden, wodurch ein Wirbelbett entsteht. Dabei wird dem Wirbelschichtreaktor Stickstoff als Wirbelgas mit einem Druck von 160 mbar zugeführt. Zum Beheizen des Wirbelschichtreaktors auf eine Betriebstemperatur von 450 °C werden mehrere Strahlheizrohre verwendet. Das Aufgabegut kann direkt in das heiße Wirbelbett gefördert werden, woraufhin die Depolymerisierung der Polymere zu dem Pyrolyseprodukt stattfinden kann. Infolge der Zersetzung der Polymere und der Verwirbelung wird der Füllstoff von dem Pyrolyseprodukt getrennt.

Der Wirbelschichtreaktor kann vorzugsweise einen Durchmesser von 450 mm und eine Höhe von 900 mm aufweisen. Die Wirbelbetthöhe kann vorzugsweise 650 mm betragen.

Ein Fluidstrom, welcher das Pyrolyseprodukt mit Methylmethacrylat umfasst, wird sodann in Schritt S41 mit einem Druck von 50 mbar aus dem Wirbelschichtreaktor abgeführt und mehreren Aufbereitungsschritten unterzogen. In den Schritten S42 und S43 wird der Fluidstrom zunächst einem Aerozyklon zum Abtrennen von Feststoffpartikeln und anschließend drei Gaswäschern zugeführt. Nachfolgend wird der Fluidstrom in Schritt S44 einem Elektroabscheider zugeführt, um besonders feine Partikel und Aerosole, die insbesondere durch die drei Gaswäscher nicht vollständig entfernt werden können, abzutrennen. In Schritt S45 wird das Pyrolyseprodukt, umfassend Methylmethacrylat, durch Kondensation als Pyrolyseöl von dem Fluidstrom abgeschieden. Das Pyrolyseöl kann im Folgenden weiter aufgereinigt werden, um Methylmethacrylat mit einer hohen Reinheit zu erhalten. Das erhaltene Methylmethacrylat kann zur Herstellung neuer Verbundkunststoffteile verwendet werden.

In Schritt S5 wird ein Teil des abgetrennten Füllstoffs gemeinsam mit einem Teil des Wirbelguts aus dem Wirbelschichtreaktor abgeführt. Dazu kann der Wirbelschichtreaktor einen Überlauf aufweisen. Der somit erhaltene Quarzsand kann ebenfalls zur Herstellung neuer Verbundkunststoffteile verwendet werden.

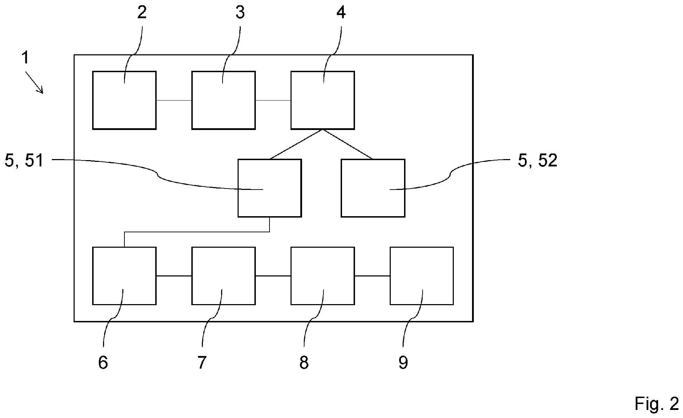

Die Figur 2 zeigt in schematischer Darstellung eine Vorrichtung gemäß einer Ausführungsform der vorliegenden Erfindung.

Die dargestellte Vorrichtung 1 zum Wiederaufbereiten von Verbundkunststoffteilen, beispielsweise aus aushärtbaren Gießmassen hergestellte Formteile wie Sanitärbecken, umfassend zumindest einen Füllstoff und zumindest ein Polymer, insbesondere Polymethylmethacrylat, insbesondere mit dem Verfahren gemäß der in Figur 1 gezeigten Ausführungsform der vorliegenden Erfindung, weist eine Zerkleinerungseinrichtung 2 zum Zerkleinern der Verbundkunststoffteile zu einem Aufgabegut und eine Zuführeinrichtung 3 zum Zuführen des Aufgabeguts in einen Wirbelschichtreaktor 4 mit zumindest einem Wirbelgut auf.

Die Vorrichtung 1 umfasst den Wirbelschichtreaktor 4 zum Depolymerisieren des zumindest einen Polymers des Aufgabeguts mittels Pyrolyse zu einem Pyrolyseprodukt, insbesondere umfassend Methylmethacrylat, und zum Trennen des Füllstoffs von dem Pyrolyseprodukt. Dabei sind der abgetrennte Füllstoff und das Wirbelgut in Material und/oder Größenverteilung im Wesentlichen ähnlich, insbesondere gleich, ausgebildet.

Eine Abführeinrichtung 5 mit zwei Abführeinheiten 51, 52 ist an den Wirbelschichtreaktor 4 angeschlossen. Dabei ist eine Abführeinheit 51 ausgebildet, einen Fluidstrom, umfassend das Pyrolyseprodukt, aus dem Wirbelschichtreaktor 4 abzuführen. Die andere Abführeinheit 52 ist ausgebildet, zumindest einen Teil des abgetrennten Füllstoffs gemeinsam mit zumindest einem Teil des Wirbelguts aus dem Wirbelschichtreaktor 4 abzuführen.

Ferner umfasst die Vorrichtung 1 einen als Aerozyklon ausgebildeten Fliehkraftabscheider 6, drei Gaswäscher 7, einen Elektroabscheider 8 sowie eine Kondensierungseinrichtung 9, die ausgebildet ist, das Pyrolyseprodukt durch Kondensation als Pyrolyseöl von dem Fluidstrom abzuscheiden.

Zusammenfassend kann zumindest eine Ausführungsform der vorliegenden Erfindung zumindest eines der folgenden Merkmale aufweisen und/oder kann zumindest einen der folgenden Vorteile bereitstellen:
- Zeit- und kosteneffizienteres Verfahren.
- Energieeffizienteres Verfahren.
- Reduzierung des Zerkleinerungsaufwands.
- Einfache und schnelle Zuführung des Aufgabeguts.
- Reduzierung der Wahrscheinlichkeit unerwünschter chemischer Reaktionen.
- Reduzierung von Verschleiß und Verschmutzung.

Obwohl die vorliegende Erfindung anhand bevorzugter Ausführungsbeispiele beschrieben wurde, ist sie nicht darauf beschränkt, sondern auf vielfältige Weise modifizierbar.

### Bezugszeichenliste

- 1: Vorrichtung
- 2: Zerkleinerungseinrichtung
- 3: Zuführeinrichtung
- 4: Wirbelschichtreaktor
- 5: Abführeinrichtung
- 6: Fliehkraftabscheider
- 7: Gaswäscher
- 8: Elektroabscheider
- 9: Kondensierungseinrichtung

- 51, 52: Abführeinheiten

- S1-S5: Schritte eines Verfahrens
- S31-S32: Schritte eines Verfahrens
- S41-S45: Schritte eines Verfahrens

## Patentansprüche

1. Verfahren zum Wiederaufbereiten von Verbundkunststoffteilen, beispielsweise aus aushärtbaren Gießmassen hergestellte Formteile, wie Sanitärbecken, umfassend zumindest einen Füllstoff und zumindest ein Polymer, insbesondere Polymethylmethacrylat, mit den Schritten:
- Zerkleinern (S1) zumindest eines Verbundkunststoffteils zu einem Aufgabegut,
- Zuführen (S2) des Aufgabeguts in einen Wirbelschichtreaktor (4) mit zumindest einem Wirbelgut,
- Depolymerisieren (S3, S31) des zumindest einen Polymers des Aufgabeguts mittels Pyrolyse zu einem Pyrolyseprodukt, insbesondere umfassend Methylmethacrylat,
- Trennen (S3, S32) des Füllstoffs von dem Pyrolyseprodukt, wobei der abgetrennte Füllstoff und das Wirbelgut in Material und/oder Größenverteilung im Wesentlichen ähnlich, insbesondere gleich, ausgebildet sind,
- Abführen (S41) eines Fluidstroms umfassend das Pyrolyseprodukt aus dem Wirbelschichtreaktor (4), und
- Abführen (S5) zumindest eines Teils des abgetrennten Füllstoffs gemeinsam mit zumindest einem Teil des Wirbelguts aus dem Wirbelschichtreaktor (4).

2. Verfahren nach Anspruch 1, **dadurch gekennzeichnet, dass** zumindest ein Teil des Aufgabeguts eine Partikelgröße von zumindest 1 µm, vorzugsweise zumindest 0,1 mm, insbesondere zumindest 1 mm, und maximal 10 mm, vorzugsweise maximal 7 mm, insbesondere maximal 5 mm, aufweist, insbesondere wobei das Aufgabegut dem Wirbelschichtreaktor (4) mittels zumindest eines Schneckenförderers zugeführt (S1) wird.

3. Verfahren nach einem der Ansprüche 1-2, **dadurch gekennzeichnet, dass** zumindest ein Teil des Aufgabeguts eine Partikelgröße zwischen 50 mm und 100 mm aufweist, insbesondere wobei das Aufgabegut dem Wirbelschichtreaktor (4) über zumindest eine Doppelklappenschleuse zugeführt (S1) wird.

4. Verfahren nach einem der Ansprüche 1-3, **dadurch gekennzeichnet, dass** eine Betriebstemperatur des Wirbelschichtreaktors (4) zwischen 400 °C und 650 °C, vorzugsweise zwischen 425 °C und 500 °C, insbesondere 450 °C, beträgt.

5. Verfahren nach einem der Ansprüche 1-4, **dadurch gekennzeichnet, dass** der Wirbelschichtreaktor (4) mithilfe zumindest eines Strahlheizrohres beheizt wird.

6. Verfahren nach Anspruch 5, **dadurch gekennzeichnet, dass** das zumindest eine Strahlheizrohr zumindest teilweise mit zumindest einem Überschussgas aus der Pyrolyse betrieben wird, insbesondere wobei zumindest eines des zumindest einen Überschussgases von dem Fluidstrom abgeschieden wird.

7. Verfahren nach einem der Ansprüche 1-6, **dadurch gekennzeichnet, dass** dem Wirbelschichtreaktor (4) zur Erzeugung eines Wirbelstroms ein Wirbelgas, insbesondere ein Inertgas, wie beispielsweise Stickstoff, mit einem Druck zwischen 140 mbar und 180 mbar, vorzugsweise von 160 mbar, zugeführt wird.

8. Verfahren nach einem der Ansprüche 1-7, **dadurch gekennzeichnet, dass** der Füllstoff und das Wirbelgut zumindest teilweise, insbesondere vollständig, anorganisches Material, beispielsweise Quarzsand, aufweisen.

9. Verfahren nach einem der Ansprüche 1-8, **dadurch gekennzeichnet, dass** der Fluidstrom mit einem Druck zwischen 40 mbar und 60 mbar, vorzugsweise von 50 mbar, aus dem Wirbelschichtreaktor (4) abgeführt (S41) wird.

10. Verfahren nach einem der Ansprüche 1-9, **dadurch gekennzeichnet, dass** der Fluidstrom zumindest einem Fliehkraftabscheider (6), insbesondere Aerozyklon, zum Abtrennen von Feststoffpartikeln zugeführt (S42) wird.

11. Verfahren nach einem der Ansprüche 1-10, **dadurch gekennzeichnet, dass** der Fluidstrom, insbesondere nach dem Zuführen zu dem zumindest einen Fliehkraftabscheider (6), zumindest einem, vorzugsweise drei Gaswäschern (7) zugeführt (S43) wird.

12. Verfahren nach einem der Ansprüche 1-11, **dadurch gekennzeichnet, dass** der Fluidstrom, insbesondere nach dem Zuführen zu dem zumindest einen Gaswäscher (7), zumindest einem Elektroabscheider (8) zugeführt (S44) wird.

13. Verfahren nach einem der Ansprüche 1-12, **dadurch gekennzeichnet, dass** das Pyrolyseprodukt durch Kondensation als Pyrolyseöl von dem Fluidstrom abgeschieden (S45) wird.

14. Verfahren nach einem der Ansprüche 1-13, **dadurch gekennzeichnet, dass** der Fluidstrom in einem Kreislauf geführt wird, wobei der Fluidstrom nach dem Abscheiden des Pyrolyseprodukts wieder dem Wirbelschichtreaktor (4) zugeführt wird.

15. Vorrichtung (1) zum Wiederaufbereiten von Verbundkunststoffteilen, beispielsweise aus aushärtbaren Gießmassen hergestellte Formteile, wie Sanitärbecken, umfassend zumindest einen Füllstoff und zumindest ein Polymer, insbesondere Polymethylmethacrylat, insbesondere mit einem Verfahren gemäß einem der Ansprüche 1 bis 14, umfassend
eine Zerkleinerungseinrichtung (2) zum Zerkleinern (S1) der Verbundkunststoffteile zu einem Aufgabegut,
eine Zuführeinrichtung (3) zum Zuführen (S2) des Aufgabeguts in einen Wirbelschichtreaktor (4) mit zumindest einem Wirbelgut,
den Wirbelschichtreaktor (4) zum Depolymerisieren (S3, S31) des zumindest einen Polymers des Aufgabeguts mittels Pyrolyse zu einem Pyrolyseprodukt, insbesondere umfassend Methylmethacrylat, und Trennen (S3, S32) des Füllstoffs von dem Pyrolyseprodukt, wobei der abgetrennte Füllstoff und das Wirbelgut in Material und/oder Größenverteilung im Wesentlichen ähnlich, insbesondere gleich, ausgebildet sind, und
eine Abführeinrichtung (5, 51, 52) zum Abführen (S41) eines Fluidstroms umfassend das Pyrolyseprodukt aus dem Wirbelschichtreaktor (4) und zum Abführen (S5) zumindest eines Teils des abgetrennten Füllstoffs gemeinsam mit zumindest einem Teil des Wirbelguts aus dem Wirbelschichtreaktor (4).
